Europäisches Patentamt

(19) European Patent Office

Office européen des brevets

(11) Publication number : **0 342 586 B1**

(12) **EUROPEAN PATENT SPECIFICATION**

(45) Date of publication of patent specification :
15.11.95 Bulletin 95/46

(51) Int. Cl.⁶ : **G01N 33/52,** G01N 33/542,
G01N 33/543

(21) Application number : **89108751.2**

(22) Date of filing : **16.05.89**

(54) **Assay device with gradientregulated localized signal.**

(30) Priority : **17.05.88 US 194702**

(43) Date of publication of application :
**23.11.89 Bulletin 89/47**

(45) Publication of the grant of the patent :
**15.11.95 Bulletin 95/46**

(84) Designated Contracting States :
**DE FR GB IT**

(56) References cited :
EP-A- 0 176 357
EP-A- 0 212 670
US-A- 3 692 486

(73) Proprietor : **ENZO BIOCHEM, INC.**
**345 Hudson Street**
**New York, N.Y. 10013 (US)**

(72) Inventor : **Rabbani, Elazar**
**69 Fifth Avenue**
**New York, NY 10003 (US)**
Inventor : **Stavrianopoulos, Jannis G.**
**1880 Riverside Drive,**
**Apt. 8E**
**New York, NY 10033 (US)**
Inventor : **Kline, Stanley**
**235 Lincoln Place**
**New York, NY 11217 (US)**
Inventor : **Hurley, Ian**
**174 Castleton Ave.,**
**Apt. 2-9**
**Staten Island, NY 10301 (US)**

(74) Representative : **VOSSIUS & PARTNER**
**Postfach 86 07 67**
**D-81634 München (DE)**

## Description

This invention relates to the field of analytical elements or devices useful for testing fluid samples and methods of their use. More particularly, it relates to elements or devices used in the testing of biological or other fluids for clinical diagnostic or research purposes.

Analytical elements or test devices have been developed which are useable by untrained individuals and do not require accompanying instrumentation. Certain of these have been described in the patent literature to provide a component or plurality of segments which have different degrees of responsiveness to samples being tested. The patents described below are exemplary.

U.S. Patent No. 3,964,871 discloses a stoichiometric antagonist reagent which reacts with as much of the final product as will exhaust the amount of the antagonist.. That is, that the antagonist will prevent in some manner the accumulation of oxidized indicator until such time as the antagonist or indicator have been completely consumed in the reaction. Preferred devices are those which have a plurality of indicator reagent sites separated from each other by a non-absorbent or hydrophobic material and each site preferably will absorb nearly identical volumes of the solvent employed in depositing reagent and of the biological fluid to be tested.

U.S. Patent No.4, 654, 310 discloses a support matrix divided into a plurality of test portions. Each test portion has the same concentration of a first catalyst reactive with the analyte and a competing second catalyst reactive with the analyte which is present in a different concentration in each portion. One catalyst gives a detectable response and the other does not. For example, peroxidase and catalase compete for peroxide which is generated. Since the ratios are different in each portion, in test portions where the ratio of indicator catalyst to non-indicator catalyst is high, the result of the indicator reaction is detectable, and in test portions where the ratio is low, the result of the indicator reaction is not detectable. The concentration of analyte in the sample can be estimated by observing the point at which changeover from detectable to non-detectable indicator reaction occurs.

U.S. Patent No. 3,723,064 discloses a layered testing device having a first porous layer impregnated with a reagent that reacts with the analyte to produce a product, a membrane adjacent to the first porous layer which has plural regions each having a different permeability to the product and containing an indicator for providing a visual indication of any of the end product reaching the indicator layer.

EP-A 212 670 discloses a method for detecting an analyte moiety by means of a localized signal. The signal is generated in a specific, indicative, viscous or thick medium comprising material which limits the spread of the signal from the point of origin of the signal.

Other devices of the prior art employ a single reagent which is sensitive to glucose above a specific concentration. These latter devices, as represented by the devices disclosed in U.S. Pat. No. 2,893,844, also give the operator a visual indication by color formed upon reaction of the reagent element with glucose. The devices do not indicate a range of concentrations, but only indicate if a certain predetermined level of glucose is present or exceeded in the solution being tested.

Although the above developments in the field of analytical devices have offered certain advantages, none of these has contemplated the complementary advantages to be realized from localization of the signal, by a localization medium, which is produced in conjunction with a gradient to enable visualization of a quantitative or semi-quantitative response.

The present invention provides an analytical device for quantitatively detecting the presence of an analyte in a sample. In contrast with analytical elements and devices previously known or described, the device of the present invention includes a signal localization component in a portion of the device combined or in contact with a gradient of a species or material which interacts with the analyte or a signal precursor produced therefrom to thereby regulate production of a detectable signal on or in the device This provides a device which is particularly suitable for easy but reliable use quantitative determinations even by untrained personnel, such as for the at-home testing of body fluid constituents which are indicative of some physical condition of the individual whose body fluid is tested.

Thus, the present invention provides a device for quantitatively detecting the presence of an analyte in a sample, which device comprises:

    a) a first reaction matrix containing therein:

        i) a first composition which produces, when exposed to the analyte of interest, an analyte derivative which is diffusible through said first reaction matrix, and

        ii) a gradient of a species which is reactive, in said first reaction matrix, with said analyte derivative to regulate the production of a detectable signal therefrom, and

    b) at least one additional matrix, in fluid contact with the gradient component of said first reaction matrix, and containing therein:

        i) a signal generating composition positioned to be contacted sequentially by said analyte derivative after contact with said gradient species, and wherein said composition is reactive with said analyte derivative to produce a detectable signal, and

ii) a signal localization component, in at least a portion of said additional matrix, comprising a solution and a localization medium or network, whereby the network is a continuous or discontinuous polymeric network which is suspended or dissolved in an aqueous or organic solution, or whereby the network is a material that imparts-a viscosity of at least 1.5 MPa.s to the medium, in which the signal is generated in said solution unbound to said localization medium and wherein the convection and diffusion of the signal in said solution is diminished by the effect of said localization medium on said solution.

Another aspect is a competitive immunoassay test device which can give a quantitative signal because of the presence of a signal regulatory gradient layer. The device has a first layer comprising a porous matrix having incorporated therein a known amount of an antibody to the analyte of interest which has been complexed with a labelled analyte or analyte analog, e.g. digoxin covalently linked to 8-(1,6- diaminohexyl)-NAD$^+$. The device has a second layer comprising a porous matrix having incorporated therein a gradient of antibody to the analyte of interest. The device has a third layer which comprises a porous matrix having a signal generating composition incorporated therein, for example E. coli homoserine dehydrogenase, homoserine nitrotetrazolium blue and diaphorase. In operation, analyte is spotted onto the first layer in which the labelled analyte is is liberated from antibody complex by analyte and permitted to diffuse through the regulatory gradient layer. Labelled analyte or analyte analog which has entered into the third layer is indicated by formation of a detectable signal, such as the occurance of a blue precipitate of formazin.

The signal remains substantially localized because it is generated in a medium that comprises a material that limits the spread of the signal from the point of origin of the signal. The media that are preferred for practicing this aspect of the present invention comprise a solution and a network or a component that imparts the desired viscosity to the medium. The network or viscous component limits the spread of the signal from the point of origin of the signal. The network is suspended or dissolved in the solution, while the viscous component is dissolved in the solution. The signal is generally a fluorescent or chromophoric compound. Localization of the signal provides the assay with increased sensitivity.

The material which is most appropriate for use as the species that is reactive with the analyte or analyte derivative to prevent production of signal therefrom along the gradient of such reactive species will depend upon the analyte being tested for and the reagent composition being used for potential production of signal. It can be an antagonist of the analyte or analyte derivative or a competitor with a component of the signal generating composition for either the analyte or analyte derivative.

The gradient can be a concentration gradient in at least a portion of the matrix. The concentration gradient can be a continuous gradient which provides an increasing amount of the reactive species along an axis defined by the gradient or the gradient can be a stepped gradient of sequentially increasing amounts of the reactive species along the axis.

The detectable signal can result from the production of a change in resistance, from the production of a charge transfer complex, from a change in color, fluorescence luminescence or any of a wide variety of known signalling systems.

The reagents for signal generation can be combined in a single layer or allocated to separate layers which are in fluid contact. In the first aspect it can be highly desirable to link the reagents together, such as where they are sequential enzymes in a reaction pathway.

The analyte moiety is the substance that is to be detected in the assay. Detection is by means of an analyte-specific moiety that, for example, forms a complex with the analyte moiety, for example an enzyme/substrate or antigen/antibody complex. Analyte moieties include, but are not limited to , microorganisms, fungi, algae, plant cells, animal cells, tumor cells, ligands, polysaccharides, polypeptides, nucleic acids, polynucleotides, drugs and normally occuring components of body fluids. The analyte moiety can be derived from serum, tissue extracts, cell smears, urine, sputum, feces, saliva, puss, semen, fermentation broth, culture media, water aliquots, environmental samples, and foods.

One embodiment of the matrix of the device comprises a network and a solution. The term network refers to a three-dimensional shaped structure pervading the medium. The term solution refers to the liquid present in the medium.

The network can be continuous or discontinuous and is formed as a result of the presence of polymers in the solution. A continuous network is present when the network is comprised of one integral polymer. A discontinuous network unit is present when the network is comprised of more than one integral polymer. The monomers in each integral polymer are linked covalently.

Polymers that provide a network can be comprised of organic monomers, inorganic monomers, or a combination of organic and inorganic monomers. The polymer can be linear or branched. Large polymers can be ground to any shape, although the bead shape is preferred. The bead can be spherical, and can be porous or non-porous. Particularly preferred is the spherical porous bead. The polymer can be suspended or dissolved in the solution.

Networks comprising polymers containing organic monomers that generally are suspended in the solution include, for example, polysaccharides, starches, liposaccharides, methylcelluloses, mucilages, plant gums, celluloses, agaroses and sepharoses. Preferred are the agarose, sepharose, and dextran polymers. Polymers containing inorganic monomers that are generally suspended in the solution include, for example, zeolites, silicates, phosphates, sulfates, and aluminates. Preferred are the silica and alumina polymers.

Networks comprising polymers containing organic monomers that generally are dissolved in the solution include, for example, carboxylmethyl cellulose, polyvinyl alcohol, polyglycols, glycols, proteins, polypeptides, pectins, mucins, lipoproteins, DNA, RNA, and polynucleotides. Preferred polymers are the carboxymethylcelluloses, polyglycols, proteins, polypeptides, DNA, RNA, polynucleotides, mucins and pectins.

Polymers that contain organic and inorganic monomers that are generally dissolved in the solution include, for example, polymers comprising bifunctional organic monomers cross-linked by bivalent metals. The bifunctional groups include, for example, aldehydes, ketones, alcohols, carboxylic acids, amines, amides, and thiols.

When the network comprises a polymer suspended as a solid in the solution, the solid network provides the barrier which reduces the bulk movement of the solution. When the network comprises a polymer dissolved in the solution, the network that provides the barrier which reduces the bulk movement of the solution arises from the non-covalent association, interaction, linking, and binding of the dissolved polymers to each other. The non-covalent interaction can be by, for example, hydrogen bonding, ion pairing, or ionic attraction.

It is believed that the network can further localize the signal by interacting with the signal. The interaction can be non-covalent or covalent. Non-covalent interaction is generally by electrostatic interaction, which includes, for example, hydrogen bonding, ion pairing, dipole-dipole interaction, and Van der Waals attraction. Covalent interaction is by the formation of a bond between the network and the signal.

Networks that can interact non-covalently with the signal comprise the various polymers containing functional groups or polymers coated with a compound containing functional groups. They include, for example, those used for normal phase chromatography such as alumina, silica gel, and carbohydrates, those used for reverse phase chromatography, such as octadecyl- and phenyl-bonded silica, and those used for gas chromatography, for example, diatomaceous earth coated with silicone oil or polyethelene glycol esters. An example of non-covalent interaction between the network and the signal is the network comprised of octadecyl-bonded silica and the signal is a phenylene diamine. The organic octadecyl groups bind the organic o-phenylene diamine molecule non-covalently, providing additional signal localization.

Networks that can interact covalently with the signal comprise, for example, those that contain a functionality that can react with a signal, for example, as by the formation of a Schiff base. An example of covalent interaction between the network and the signal is where the precursor used in an assay is an aromatic dye comprising an alpha diphosphorylated gem diol, the network in the medium is a polymer comprising a primary amino group, and the signalling moiety attached to the analyte-specific moiety is alkaline phosphatase. The cleavage of the phosphate groups from the gem diol, by the enzyme eliminates a molecule of water and forms an aldehyde-containing fluorescent signal. The aldehyde group on the signal can react with amino group on the network to form a Schiff base, thus immobilizing the signal to the network, and providing additional signal localization.

This invention also contemplates signal generating assays in a medium on a support wherein the support interacts with the signal and provides additional signal localization. The interaction can be covalent or non-covalent as described hereinabove wherein the network interacts with the signal.

The solution component of the medium can be any liquid compatible with the particular assay and the network. Aqueous solutions or organic solutions can be used for signalling moieties which are catalysts, for example, enzymes. See Enzymatic Catalysis In Organic Media at 100°C, A. Zaks and A.M. Klibanov, Science. 224, 1249-1251 (1984), and Preparative Production of Optically Active Esters and Alcohols using Esterase-Catalyzed Stereospecific Transesterification In Organic Media by B. Cambuv and A. M. Klibanov, J. Amer. Chem. Soc. 106, 2687-2692 (1984) which are hereby incorporated by reference. In other instances certain organic solvents can be useful to accelerate the reaction by solubilizing the analyte. See Randolph, et al, Science 238:387-390 (1988).

The method of this invention is adaptable to both precipitable and soluble assays. For precipitable assays, the reduction in spread by the network allows the signal to concentrate in a small volume, i.e., signal localization, and thus exceed the signal solubility in the solution so that a visible precipitate is formed. For soluble assays, the reduction in spread by the network allows the signal molecules to remain concentrated so that the signal, for example, fluorescence, luminescence, or color can be more readily observed.

In one embodiment, the medium or matrix of the present invention is prepared by suspending or dissolving in an aqueous or organic solution the polymer that forms the network. At times, it may be necessary to heat the medium and then cool it to ensure uniform network distribution. The medium can optionally further comprise a buffer.

The concentration of the network in the solution required by this invention depends on the particular network and the particular solution. For polymeric networks which are suspended in the solution, the amount of solution present will be dictated, for example, by the size, shape, and swelling of the polymer. The swelling of the polymer is generally controlled by the percent of cross-linkage, with the swelling being inversely proportional to the percent of cross-linkage. Highly cross-linked polymers contain high ratios of network to solution, as compared to low cross-linked polymers and thus form tight beads. Tighter beads can be advantageous in reducing spread of a signal because they can provide a dense network layer. Low cross-linked polymers contain high ratios of solution to network as compared to highly cross-linked polymers and form soft beads which are advantageous when large amounts of solution are desired, for example, when the precursor is relatively insoluble in the solution. Preferred suspended networks are gels. Tables for determining the correlation between cross-linkage and swelling of the polymer are readily available. See the pamphlets Ion-Exchange Chromatography, and Gel Filtration Chromatography, by Pharmacia Chemical Company, Piscataway, New Jersey.

For polymeric networks which are dissolved in the solution, the polymer should generally comprise from about one percent to about fifty percent, preferably, from about two percent to about thirty percent, and more preferably, from about five percent to twenty percent of the solution (weight/volume). High concentrations of polymer provide a very elaborate network and can significantly reduce the spread of signal as compared to low concentrations of polymers. However, high concentrations of polymers can make the medium very viscous, thus making it difficult to pour or layer the medium over a support. Thus, optimum concentration of the polymers in the solution will thus depend on the type of assay to be carried out. This is best determined by preliminary control experiments which an be readily carried out by those skilled in the art.

An example of a medium where the network is formed by a dissolved polymer is where 2% carboxylmethyl-cellulose (cmc) (w/v) is heated in water to about 50°C to form a clear medium, and the medium is then permitted to cool. The medium remains clear, and the soluble network is uniformly dispersed in the medium. An example of a medium where the network is formed by a suspended polymer is where 2% agarose is heated in water to about 60°C to form a clear medium, and the medium is then permitted to cool. Upon cooling, the swollen agarose separate, from and settle, out of the water as beads. The swollen beads and the water entrapped in and between the beads form a medium comprising an insoluble network.

A second embodiment of the matrix materials which form the device of the present invention relates to material that imparts a viscosity of at least 1.5 centipoise (cp) to the medium. The term solution refers to the solvent and other liquids in the medium. When the signal is a chromogenic or fluorescent compound, reducing the diffusion of this signal by increasing the viscosity of the solution will result in localization of the signal in one area and will enhance its detection.

The viscous component can be any molecule that is soluble in the solution and able to increase the viscosity of the medium. A viscous or thick medium, for the purpose of this invention, is defined as one having a viscosity of at least 1.5 cp. The viscosity of the medium can, however, range from about 1.5 cp to about 1,000 cp. The diffusion of a signal is not significantly retarded in a medium whose viscosity is lower than about 1.5 cp. A medium whose viscosity is greater than about 1000 cp may hinder the diffusion of analyte or analyte derivative and may not be capable of being layered or poured over the support. However, media with a viscosity higher than 1000 cp can be used if the medium can be layered over the support and the analyte or analyte derivative can diffuse to the signal generating composition Viscosity is dependent on the temperature. It is contemplated that the viscosity of the medium as claimed by this invention is ascertained at the time when the signal is generated in the medium. Thus, whether the assay is carried out at room temperature, 37°C, at 0°C, or any other temperature, it is at this temperature at which the viscosity of the medium should be at least about 1.5 cp and preferably form about 1.5 cp to about 1000 cp.

The viscous component should not materially affect the reaction of the reactive species of the gradient or the signal generating system with the analyte or analyte derivative.

The concentration of the viscous component required to produce a viscosity of about 1.5 cp or higher will vary with the particular viscous component. There are tables available which disclose the viscosities of solutions containing varying concentrations of different components. See for example Bingham and Jackson, Bureau Standards Bulletin, 14, 59 (1918), M.L. Sheeley, Ind. Eng. Chem., 24, 1060 (1932), and Segur and Oberstar, Ind. Eng. Chem., 43, 2117 (1951). For other components that are useful in increasing the viscosity of solution, the amount of required material can be readily determined by one skilled in the art by gradually adding the material until the desired viscosity, as measured, for example, by a viscometer, is obtained.

Suitable viscous components include monomers, oligomers, and polymers soluble in the solution. For example, sugars, alcohols, glycols, proteins, polypeptides, amino acids, deoxynucleic acids, ribonucleic acids, polynucleotides, nucleotides, nucleosides, bases, fatty acids, pectins, mucins, polysaccharides, steroids, flavanoids, alkaloids, terpenes, vitamins, co-enzymes, and prostaglandins, hydrocarbons, aromatic hydrocarbons, aromatic hydrocarbons, and porphyrins.

Preferred viscous components include, for example, sugars glycols, proteins, polypeptides, amino acids, deoxynucleic acids, ribonucleic acids, polynucleotides, nucleotides, nucleosides, bases, pectins, mucins, and polysaccharides.

Some specific components in addition to retarding diffusion of the signal by increasing the viscosity of the medium, can also decrease spread through convection by forming a network in the medium. An example of such a component is carboxymethylcellulose.

The specific component retards the diffusion of the signal by increasing the shear gradient or shear rate of the medium which in effect serves to increase the viscosity of the medium. The extent of increase in the viscosity of the medium by the specific component depends on the axial ratio, shape, and size of the specific component. The viscosity of a medium comprising a specific component can be determined by methods known in the art. Such methods include, for example, an Ostwald capillary viscometer, a Ubbelohde capillary viscometer, a Couette viscometer, a Zimm-Crothers floating-rotor viscometer, and a Cartesian-diver rotating-cylinder viscometer. See for example Physical Biochemistry by David Freifelder, pp. 359-363.

The viscous matrix can be prepared by suspending or dissolving in an aqueous or organic solution, an amount of the component that produces a viscosity of at least about 1.50 cp. For some rather insoluble components, it may be necessary to initially heat the solution and then permit it to cool in order to ensure uniform component distribution. The matrix can optionally further comprise a buffer.

Matrices of high viscosity will be more effective in localizing a signal than those of low viscosity; but an overly high viscosity may retard the diffusion of the analyte or analyte derivative, and thus prevent the signalling moiety from rapidly contacting and reacting with the signal precursor. Thus, the actual viscosity chosen for the medium depends on the particular assay. The optimum viscosity for a particular assay can readily be determined by a person skilled in the art using standard methods.

The solution part of the medium can be any liquid compatible with the analyte, analyte derivatives, signal generating composition and the reactive species of the gradient. Aqueous solutions or organic solutions can be used for signal components which are catalysts, for example, enzymes. In some instances organic solutions are preferred. See Enzymatic Catalysis In Organic Media at 100°C, A. Zaks and A.M. Klibanov, Science. $\underline{224}$, 1249-1251 (1984), and Preparative Production of Optically Active Esters and Alcohols using Esterase-Catalyzed Stereospecific Transesterification In Organic Media by B. Cambuv and A.M. Klibanov, J. Amer. Chem, Soc, $\underline{106}$, 2687-2692 (1984). Certain organic solvents can be useful to accelerate the reaction by solubilizing the analyte. For example such organic solvents are advantageous in solubilizing bound cholesterol.

## SIGNAL GENERATING COMPOSITION

The term "signal generating composition" refers to a signal precursor and those additional conversion reagents necessary to convert the signal precursor to produce a detectable species therefrom in response to the presence of the analyte or an analyte derivative.

If the analyte derivative is hydrogen peroxide, and the signal converter is a peroxidase, the signal precursor can be, for example, 4-chloronaphtol, tetramethylbenzidine, amino-antipyrine and a phenol, starch and potassium iodide or orthophenylenediamine.

If the analyte derivative is iodine, and the signal converter is starch, the signal precursor can be, for example, potassium iodide.

If the analyte deriative is NADH or NADPH, and the signal converter is phenazine methosulfate or diaphorase, the signal precursor can be, for example, any of a variety of tetrazolium salts.

If the analyte derivative is an antigen covalently linked to NAD or NADP, and the signal converter is an NAD or NADP dependent oxido-reductase enzyme and its substrate in addition to phenazine methosulfate or diaphorase, the signal precursor can be, for example, any of a variety of tetrazolium salts.

If the analyte derivative is iodine, the signal precursor can be of polyacetylene. The signal will consist of an increase in conductivity of the polyacetylene.

The method of this invention is adaptable to both precipitable and soluble assays. For precipitable assays, the retardation in diffusion allows the signal to concentrate in a small volume and thus exceed their solubility in the solution so that a visible precipitate is formed. For soluble assays, the retardation in diffusion allows the signal molecules to remain concentrated so that fluorescence, luminescence, or chromogen can be more readily observed.

SIGNAL REGULATORY GRADIENT

The material which is most appropriate for use as the species that interacts with the analyte or analyte derivative (the regulatory species) to prevent production of signal therefrom along the gradient of such regulatory species will depend upon the analyte being tested for and the reagent composition being used for potential production of signal.

If the analyte is an antigen, and the analyte specific moiety is an antibody to that antigen complexed to the antigen covalently labeled with NAD or NADP, and the analyte derivitive is the antigen covalently labeled with NAD or NADP, the regulatory or interactive species can be the antibody to the antigen.

If the analyte is cholesterol the regulatory or interactive species can be digitonin or tomatine.

If the analyte is cholesterol or glucose or uric acid, and the analyte specific moiety is cholesterol oxidase or glucose oxidase or uric acid oxidase, or any analyte, and the analyte specific moiety is cholesterol oxidase or glucose oxidase or uric acid oxidase or any combination of enzymes which will produce hydrogen peroxide stoichiometrically from the analyte with appropriate substrates, and the analyte derivitive is hydrogen peroxide,the regulatory or interactive species can be catalase or iodine or ferricyanide or mercurous salts or peroxidase and an oxidizable species such as phenols or polyphenols, aromatic amines or polyamines.

If the analyte is cholesterol or glucose or uric acid or any analyte, and the analyte specific moiety is cholesterol oxidase or glucose oxidase or uric acid oxidase or any combination of enzymes which will produce hydrogen peroxide stoichiometrically from the analyte with appropriate substrates, and the primary analyte derivitive is hydrogen peroxide, and the secondary analyte derivitive is iodine, the regulatory or interactive species can be an acyl hydrazide or tyrosine or polytyrosine.

If the analyte is cholesterol, and the analyte specific moiety is cholesterol dehydrogenase, and the analyte derivitive is NADH or NADPH, the regulatory or interactive species may be iodine or quinones or NADH or NADPH requiring enzymes (such as glutathione reductase).

The gradient can be a concentration gradient in at least a portion of the matrix. The gradient can be a continuous gradient which provides an increasing amount of analyte or analyte derivative reactive species along an axis defined by the gradient or the gradient can be a stepped gradient of sequentially increasing amounts of the reactive species along the axis.

The methods of the present invention will be more clearly understood from the following illustrative examples.

EXAMPLE 1

The present example describes preparation and use of a three-layer test device for cholesterol having a step gradient in accordance with the invention.

Layer 1, the bottom layer is formed from a composition comprising 4-chloronaphthol (10mM), horseradish peroxidase (1 ug/ml), bovine serum albumin (1 mg/ml), sodium acetate( pH 5.6, 0.1 M), agarose (0.7% w/v) and n-butanol (9% v/v).

Layer 2, the middle layer, is formed from a composition comprising catalase (0 to 0.16 ug/ml), sodium acetate (pH 5.6, 0.1M), agarose (0.7% w/v) and n-butanol (9% v/v)

Layer 3, the top layer, is formed from a composition comprising cholesterol oxidase (Nocardium erthropolis)(2U/ml), cholesterol esterase (bovine pancreas)(2U/ml), taurocholic acid(7.5mg/ml), bovine serum albumin(1 mg/ml), sodium acetate(pH 5.6, 0.1M), agarose(0.7% w/v), and n-butanol(9% v/v).

The composition for layer 1 (200 ul), the composition for layer 2 (400ul) and the composition for layer 3(100 ul) are added sequentially into 5 ml propylene culture tubes (11 mm in diameter). Each composition is permitted to solidify into a formed layer at 0° before the subsequent composition is added. A full set consists of 5 catalase concentrations ranging from 0 to 0.16 ug/ml

Aliquots of human serum were diluted to 50ul with 0.1M sodium acetate pH 5.6 and layered on top of the top layer. After 60 to 80 minutes the tubes were marked by the presence or absence of a blue precipitate between layer 1 and layer 2. The results are presented in TABLE I.

## TABLE I

#### Precipitate

| Tube | Catalase (ug/ml) | 5ul Serum | 10 ul Serum | 20 ul Serum |
|------|------------------|-----------|-------------|-------------|
| 1 | 0.00 | + | + | + |
| 2 | 0.02 | - | + | + |
| 3 | 0.04 | - | - | + |
| 4 | 0.08 | - | - | - |
| 5 | 0.16 | - | - | - |

The above results demonstrate regulation of signal production by the concentration gradient of catalase present in the device.

EXAMPLE 2

Cholesterol oxidation solutions having a variety of organic solvents therein were prepared as follows.
A first composition was prepared to contain:

| | |
|---|---|
| Cholesterol oxidase(Nocardia erthopolis) | 0.15 U/ml |
| Horseradish peroxidase | 0.25 ug/ml |
| amino antipyrine | 1.25 mM |
| 3, 5-dichloro-2-hydroxybenzene sulfonic acid | 10.0 mM |
| Taurocholic acid | 7.5 mg/ml |
| Sodium acetate(pH 6.0) | 0.1 M |

To form the final composition, varying amounts of aliphatic alcohols including ethanol, 1-propanol and n-butanol were added. Reaction was initiated by addition of 5ul of human serum which contained 2.4 mg/ml total(bound and unbound) cholesterol. The samples were incubated at 25° C and absorbance was monitored(500 nm) with time.

Relative oxidation rates were calculated as time required for 50% reaction. They were normalized to the oxidation solution containing no alcohol. The effect of alcohols on cholesterol oxidation by cholesterol oxidase are shown in Table II:

## Table II

| Alcohol | Relative Oxidation Rate/Concentration | | | | |
|---------|---|---|---|---|---|
| | 0 | 5 | 10 | 15 | 20 |
| ethanol | 1 | 1.1 | 1.8 | 2.1 | 1.8 |
| isopropanol | 1 | - | - | - | 9.8 |
| n-butanol | 1 | - | 7.0 | - | - |

The above results demonstrate that aliphatic alcohols enhance the rate of oxidation of cholesterol in serum.

**Claims**

1. A device for quantitatively detecting the presence of an analyte in a sample, which device comprises:
   a) a first reaction matrix containing therein:
      i) a first composition which produces, when exposed to the analyte of interest, an analyte derivative which is diffusible through said first reaction matrix, and
      ii) a gradient of a species which is reactive, in said first reaction matrix, with said analyte derivative to regulate the production of a detectable signal therefrom, and
   b) at least one additional matrix, in fluid contact with the gradient component of said first reaction matrix, and containing therein:
      i) a signal generating composition positioned to be contacted sequentially by said analyte derivative after contact with said gradient species, and wherein said composition is reactive with said analyte derivative to produce a detectable signal, and
      ii) a signal localization component, in at least a portion of said additional matrix, comprising a solution and a localization medium or network, whereby the network is a continuous or discontinuous polymeric network which is suspended or dissolved in an aqueous or organic solution, or whereby the network is a material that imparts a viscosity of at least 1.5 MPa.s to the medium, in which the signal is generated in said solution unbound to said localization medium and wherein the convection and diffusion of the signal in said solution is diminished by the effect of said localization medium on said solution.

2. The device in claim 1, wherein the gradient is a concentration gradient of reactive species, in at least a portion of the first matrix, along an axis parallel to the plane of contact of said matrices.

3. The device of any of claims 1-2 wherein said signal localization component is a polymeric network, a viscosity enhancer, or a combination of the foregoing.

4. The device of any of claims 1-2 wherein said first reaction matrix comprises a gel composition of uniform porosity.

5. The device of any of claims 1-2 wherein said first reaction matrix comprises a single gel layer.

6. The device of any of claims 1-2 wherein said first reaction matrix comprises a first gel layer having said first composition therein and, in fluid contact therewith, a second gel layer having said reactive species gradient therein.

7. The device of any of claims 1-2 wherein said gradient comprises a continuous gradient which provides an increasing amount of said reaction product reactive species along said axis.

8. The device of any of claims 1-2 wherein said gradient comprises a series of zones of sequentially increas-

9

ing amounts of said reaction product reactive species along said axis.

9. The device of any of claims 1-2 wherein said signal is a precipitable reaction product.

10. The device of any of claims 1-2 wherein said signal results from the production of a change in resistance.

11. The device of any of claims 1-2 wherein said signal results from the production of a charge transfer complex.

**Patentansprüche**

1. Vorrichtung zur quantitativen Bestimmung der Gegenwart eines Analyten in einer Probe, wobei die Vorrichtung folgende Bestandteile umfaßt:
   a) eine erste Reaktionsmatrix, enthaltend:
   i) eine erste Zusammensetzung, die, wenn sie mit dem Analyten von Interesse in Kontakt gebracht wird, ein Analytderivat erzeugt, das durch die erste Reaktionsmatrix diffundierbar ist, und
   ii) einen Gradienten einer Spezies, die zur Regulation der Erzeugung eines nachweisbaren Signales davon in der ersten Reaktionsmatrix mit dem Analytderivaten reaktiv ist, und
   b) mindestens eine zusätzliche Matrix in flüssigem Kontakt mit der Gradientenkomponente der ersten Reaktionsmatrix und enthaltend:
   i) eine signalerzeugende Zusammensetzung, die so angeordnet ist, daß sie im Anschluß an den Kontakt mit der Gradientenspezies mit dem Analytderivat in Kontakt gebracht werden kann, und wobei die Zusammensetzung mit dem Analytderivat unter Erzeugung eines nachweisbaren Signals reaktiv ist, und
   ii) eine Signallokalisierungskomponente in mindestens einem Teil der zusätzlichen Matrix, umfassend eine Lösung und ein Lokalisierungsmedium oder -netzwerk, wobei das Netzwerk ein kontinuierliches oder diskontinuierliches Polymernetzwerk ist, das in einer wäßrigen oder organischen Lösung suspendiert oder gelöst ist, oder wobei das Netzwerk ein Material ist, das dem Medium eine Viskosität von mindestens 1,5 MPa.s verleiht, wobei das Signal in der Lösung nicht gebunden an das Lokalisierungsmedium erzeugt wird und wobei die Konvektion und Diffusion des Signals in der Lösung durch die Wirkung des Lokalisierungsmediums auf die Lösung vermindert wird.

2. Vorrichtung nach Anspruch 1, wobei der Gradient ein Konzentrationsgradient einer reaktiven Spezies ist, in mindestens einem Teil der ersten Matrix, entlang einer Achse, die parallel zur Kontaktebene der Matrices ist.

3. Vorrichtung nach einem der Ansprüche 1 bis 2, wobei die Signallokalisierungskomponente ein polymernetzwerk, ein Viskositätsverstärker oder eine Kombination davon ist.

4. Vorrichtung nach einem der Ansprüche 1 bis 2, wobei die erste Reaktionsmatrix eine Gelzusammensetzung von einheitlicher Porosität umfaßt.

5. Vorrichtung nach einem der Ansprüche 1 bis 2, wobei die erste Reaktionsmatrix eine einzelne Gelschicht umfaßt.

6. Vorrichtung nach einem der Ansprüche 1 bis 2, wobei die erste Reaktionsmatrix eine erste Gelschicht mit der ersten Zusammensetzung darin umfaßt, und in flüssigem Kontakt damit eine zweite Gelschicht mit dem reaktiven Speziesgradienten darin.

7. Vorrichtung nach einem der Ansprüche 1 bis 2, wobei der Gradient einen kontinuierlichen Gradienten umfaßt, der eine steigende Menge der mit dem Reaktionsprodukt reaktiven Spezies entlang der Achse liefert.

8. Vorrichtung nach einem der Ansprüche 1 bis 2, wobei der Gradient eine Reihe von Zonen umfaßt mit kontinuierlich ansteigenden Mengen der mit dem Reaktionsprodukt reaktiven Spezies entlang der Achse.

9. Vorrichtung nach einem der Ansprüche 1 bis 2, wobei das Signal ein präzipitierbares Reaktionsprodukt ist.

**10.** Vorrichtung nach einem der Ansprüche 1 bis 2, wobei das Signal sich aus der Erzeugung einer Widerstandsänderung ergibt.

**11.** Vorrichtung nach einem der Ansprüche 1 bis 2, wobei das Signal sich aus der Erzeugung eines Ladungstransferkomplexes ergibt.

**Revendications**

**1.** Dispositif pour détecter de façon quantitative la présence d'un analyte dans un échantillon, lequel dispositif comprend:

a) une première matrice de réaction contenant dans celle-ci:

i) une première composition qui produit, lorsqu'elle est exposée à l'analyte intéressant, un dérivé de l'analyte qui peut être diffusé dans la première matrice de réaction, et

ii) un gradient d'une espèce qui est réactive, dans ladite première matrice de réaction, avec ledit dérivé de l'analyte, pour régler la production d'un signal détectable provenant de celle-ci, et

b) au moins une matrice supplémentaire, en contact fluide avec le composant de gradient de ladite première matrice de réaction, et contenant dans celle-ci:

i) une composition générant un signal, placée de façon à être touchée séquentiellement par ledit dérivé de l'analyte après contact avec ledit gradient d'espèce, et dans laquelle ladite composition réagit avec ledit dérivé de l'analyte en produisant un signal détectable, et

ii) un composant de localisation de signal, au moins dans une partie de ladite matrice supplémentaire, comprenant une solution et un support de localisation ou réseau, de façon que le réseau soit un réseau polymère continu ou discontinu, qui est en suspension ou dissous dans une solution aqueuse ou organique, ou de façon que le réseau soit un matériau qui communique au support une viscosité de 1,5 Mpa.s au moins, dans lequel le signal est généré dans ladite solution, sans lien audit support de localisation, et dans lequel la convection et la diffusion du signal dans ladite solution sont diminuées par l'effet dudit support de localisation sur ladite solution.

**2.** Dispositif selon la revendication 1, dans lequel le gradient est un gradient de concentration d'espèces réactives, au moins dans une partie de la première matrice, le long d'un axe parallèle au plan de contact desdites matrices.

**3.** Dispositif selon l'une quelconque des revendications 1-2, dans lequel ledit composant de localisation de signal est un réseau polymère, un activateur de viscosité, ou une association de ce qui précède.

**4.** Dispositif selon l'une quelconque des revendications 1-2, dans lequel ladite première matrice de réaction comprend une composition de gel de porosité uniforme.

**5.** Dispositif selon l'une quelconque des revendications 1-2, dans lequel ladite première matrice de réaction comprend une couche de gel unique.

**6.** Dispositif selon l'une quelconque des revendications 1-2, dans lequel ladite première matrice de réaction comprend une première couche de gel ayant ladite première composition dans celle-ci, et en contact fluide avec celle-ci, une seconde couche de gel ayant dans celle-ci ledit gradient d'espèce réactive.

**7.** Dispositif selon l'une quelconque des revendications 1-2, dans lequel ledit gradient comprend un gradient continu qui fournit une quantité croissante de ladite espèce réactive du produit de réaction le long dudit axe.

**8.** Dispositif selon l'une quelconque des revendications 1-2, dans lequel ledit gradient comprend une série de zones de valeurs augmentant en séquence de ladite espèce réactive du produit de réaction le long dudit axe.

**9.** Dispositif selon l'une quelconque des revendications 1-2, dans lequel ledit signal est un produit de réaction pouvant être précipité.

**10.** Dispositif selon l'une quelconque des revendications 1-2, dans lequel ledit signal provient de la production d'une variation de résistance.

11. Dispositif selon l'une quelconque des revendications 1-2, dans lequel ledit signal provient de la production d'un complexe de transfert de charges.